# EUROPEAN PATENT APPLICATION

(11) **EP 3 542 783 A1**
(43) Date of publication of application: **25.09.2019**
(21) Application number: 16921955.7
(22) Date of filing: 17.11.2016
(51) Int. Cl.: A61K 8/73, A61K 8/31, A61K 8/37, A61K 8/891, A61Q 1/02, A61Q 1/08, A61Q 1/10, A61Q 1/12

(54) **OILY COMPOSITION AND OILY COSMETIC**

(71) Applicant: Shiseido Company Ltd., Chuo-ku Tokyo 104-0061 (JP)
(72) Inventor: NARUMI, Yuko, Yokohama-shi Kanagawa 224-8558 (JP); SUZUKI, Takahiro, Yokohama-shi Kanagawa 224-8558 (JP); TAKAHASHI, Kiyoshi, Yokohama-shi Kanagawa 224-8558 (JP); HIROSAKI, Ken, Yokohama-shi Kanagawa 224-8558 (JP); IKEDA, Tomoko, Yokohama-shi Kanagawa 224-8558 (JP)
(74) Representative: Ter Meer Steinmeister & Partner
(86) International application number: PCT/JP2016/004911
(87) International publication number: WO 2018/092175

(57) **Abstract**

[Objective]

To provide an oil based composition having a smooth sensation of use, which exhibits a superior unevenness correcting effect, and which may be filled in a container which is not airtight.

[Constitution]

An oil based composition includes, with respect to 100% by mass of the oil based composition:
(a) 1 to 20% by mass of a dextrin fatty acid ester;
(b) less than 5% by mass of a volatile oil component having a boiling point of less than or equal to 250°C at normal pressure;
(c) 10 to 60% by mass of a non volatile oil which is a liquid at normal temperature and normal pressure; and
(d) 10 to 50% by mass of a silicone resin powder.

## Description

### Technical Field

The present invention is related to an oil based composition having an unevenness correcting effect, and an oil based cosmetic which is formed by filling a container with the oil based composition.

### Background Art

Oil based transparent cosmetics are mainly advantageous in the points that they exhibit an attractive appearance, a finish having transparency when applied, and the like. Therefore, various transparent base materials are being considered.

For example, Patent Literature 1 discloses transparent solid cosmetic that includes a dextrin fatty acid ester as a transparent base, a liquid oil, and amorphous fine particle anhydrous silica having a particle diameter within a range from 0.001 to 0.05 µm, and spherical anhydrous silica having a particle diameter within a range from 0.1 to 30 µm.

In addition, Patent Literature 2 discloses a transparent solid composition, in which a dextrin fatty acid ester, a volatile oil component, an oil component having a refractive index within a range from 1.4 to 1.6, and a spherical powder having a refractive index within a range from 1.3 to 1.6 and an average particle diameter within a range from 3 to 30 µm are bended, from the viewpoint of exhibiting an effect of correcting unevenness of the skin to cause pores to become inconspicuous.

Further, Patent Literature 3 discloses an oil based cosmetic for correcting unevenness that combines a spherical organo polysiloxane elastomer powder and a pearl pigment, to scatter light on the skin with the spherical powders.

### Citation List

### Patent Literature

[Patent Literature 1] Japanese Unexamined Patent Publication No. H11-255616
[Patent Literature 2] Japanese Unexamined Patent Publication No. 2005-213145
[Patent Literature 3] Japanese Unexamined Patent Publication No. 2010-163370

### Summary of the Invention

### Technical Problem

However, the transparent cosmetics in which dextrin fatty acid esters are blended as employed in Patent Documents 1 and 2 has problems such as a heavy spreading sensation at the time of application, a sensation of stickiness of oil remaining, resulting in a lack of a sensation of freshness, and poor stability over time. These problems were considered to be essentially inherent to preparations in which a dextrin fatty ester is blended as a transparent base material, and therefore difficult to solve. In addition, the oil based cosmetic for correcting unevenness disclosed in Patent Literature 3 also does not exhibit a favorable sensation of adhesion to the skin or spreading expansion properties, and there is a problem in the point of sensation of use. Further, because the compositions disclosed in Patent Documents 2 and 3 contain large amounts of powder and volatile oil components, there is a problem with respect to stability, such as powdering occurring due to volatilization of the oil components. For this reason, in the case that these compositions are employed as cosmetics, it is necessary for these cosmetic to be filled in highly airtight containers. There is demand among manufacturers from viewpoints of versatility and cost, and demand among users from the viewpoints of ease of use and the like, for a cosmetic which can be utilized in a container which is not airtight. Further, there are cases in which the skin may appear white and powdery in the case of dry skin or wintertime, and a base material which can be utilized regardless of the season or skin type (dry or oily) is demanded, from the viewpoint of lasting cosmetic effects.

The present invention has been developed in view of the foregoing problems. It is an object of the present invention to provide an oil based composition which has a fresh sensation of use (hereinafter, also referred to as fresh sensation) even though it includes a dextrin fatty acid ester, is superior in unevenness correcting effects, and may be filled in a container which is not airtight. It is another object of the present invention to provide an oil based cosmetic which is formed by filling a container with the oil based composition.

### Solution to the Problem

An oil based composition of the present invention includes, with respect to 100% by mass of the oil based composition:
(a) 1 to 20% by mass of a dextrin fatty acid ester;
(b) less than 5% by mass of a volatile oil component having a boiling point of less than or equal to 250°C at normal pressure;
(c) 10 to 60% by mass of a non volatile oil which is a liquid at normal temperature and normal pressure; and
(d) 10 to 50% by mass of a silicone resin powder.

The silicone resin powder (d) preferably contains 0.1 to 46% by mass of a silicone rubber powder (d1) with respect to 100% by mass of the oil based composition. The content of the silicone rubber powder (d1) is within a range from a 0.002% to 92% by mass with respect to 100% by mass of the silicone resin powder.

The silicone rubber powder (d1) is preferably a cross polymer that includes dimethicone.

The oil based composition of the present invention may further include (e) a spherical powder.

The oil based cosmetic composition of the present invention is formed by filling the oil based composition of the present invention in container which is not airtight.

### Advantageous Effects of the Invention

The oil based composition of the present invention includes, with respect to 100% by mass of the oil based composition:
(a) 1 to 20% by mass of a dextrin fatty acid ester;
(b) less than 5% by mass of a volatile oil component having a boiling point of less than or equal to 250°C at normal pressure;
(c) 10 to 60% by mass of a non volatile oil which is a liquid at normal temperature and normal pressure; and
(d) 10 to 50% by mass of a silicone resin powder. Therefore, the oil based composition has a fresh sensation of use even though it includes a dextrin fatty acid ester, and has an extremely superior unevenness correcting effect.

In addition, since it is possible for the oil based composition of the present invention to be filled in a container which is not airtight, a container having high versatility, cost effectiveness, etc. may be selected, and the oil based cosmetic of the present invention will also be easy for users to utilize.

### Description of Embodiments

An oil based composition of the present invention includes, with respect to 100% by mass of the oil based composition:
(a) 1 to 20% by mass of a dextrin fatty acid ester;
(b) less than 5% by mass of a volatile oil component having a boiling point of less than or equal to 250°C at normal pressure;
(c) 10 to 60% by mass of a non volatile oil; and
(d) 10 to 50% by mass of a silicone resin powder.

Here, the oil based composition refers to a composition in which the oil component is a continuous phase, and the powder is dispersed in the oil, and may include plant extract, water, and alcohol in an amount of 5% by mass or less.. In addition, the oil based composition may be a solid or a fluid.

Each of the components will be described in detail below.

### (a) Dextrin Fatty Acid Ester

The dextrin fatty acid ester is an ester of dextrin and a fatty acid having a linear or branched alkyl group (preferably having a carbon number of 3 to 30), and is obtained by reacting a fatty acid chloride with dextrin under heat, employing pyridine as a basic catalyst.

It is preferable for the dextrin fatty acid ester to be that in which dextrin is bonded with myristic acid, palmitic acid, palmitoleic acid, stearic acid, isostearic acid, oleic acid, linoleic acid, linolenate, arachic acid, hexanoic acid, decanoic acid, and these fatty acids having substituent groups such as branches, hydroxyl groups and phenyl groups.

Dextrin palmitate is particularly preferable.

The amount of dextrin fatty acid ester which is blended is within a range from 1 to 20% by mass and preferably a range from 7 to 14% by mass with respect to 100% by mass of the oil based composition. By setting the amount of the dextrin fatty acid ester to be blended to be within the above range, it is possible to realize favorable moldability, stability, usability, and finish (hard and not possible to be taken up if the amount is excessively great, excessively soft, unstable, and not possible to adhere to the skin if the amount is excessively small). Although the amount of the dextrin fatty acid ester which is blended varies according to the types and the amounts of other components to be blended, a preferable amount of the dextrin fatty acid ester which is blended for a composition which is a fluid is greater than or equal to 1% by mass and less than 3% by mass, and a a preferable amount of the dextrin fatty acid ester which is blended for a composition which is a solid is greater than or equal 3% by mass. Here, a solid refers to a state in which there is no fluidity at a normal temperature (15 to 25°C) and a normal pressure.

### (b) Volatile Oil Component having a Boiling Point of Less Than or Equal to 250°C at Normal Pressure

Examples of volatile oils having a boiling point of less than or equal to 250°C at normal pressure include isoparaffin (approximately 225°C); cyclic polysiloxanes such as octamethylcyclotetrasiloxane (175°C), decamethyl cyclopentasiloxane (210°C), dodecamethyl cyclopentasiloxane (245°C); dimethylpolysiloxane having a boiling point of less than or equal to 250°C, and derivatives thereof. A cyclic polysiloxane is preferable.

The amount of the volatile oil component having a boiling point of less than or equal to 250°C at normal pressure to be blended is less than 5% by mass, preferably less than 3% by mass, and more preferably less than 1% by mass with respect to the total amount of the composition. It is possible for the volatile oil component to not be included in the composition. By the amount of the volatile oil component to be blended being less than 5% by mass, it will be possible for the oil based composition which to be filled in a container which is not airtight.

### (c) Non Volatile Oil which is a Liquid at Normal Temperature and Normal Pressure

The non volatile oil component is a liquid oil that does not exhibit volatility at normal temperature (25°C) and normal pressure. Examples of the non volatile oil component include a hydrocarbon oil, an ester oil, a silicone oil, an ultraviolet ray absorbing agent, and the like. The content thereof is 10 to 60% by mass with respect to the total amount of the composition. By the content of the non volatile oil component being greater than or equal to 10% by mass, the composition can be appropriately molded, and can be easily applied to the skin with a finger. In addition, by the content of the non volatile oil component being less than or equal to 60 % by mass, a sensation of use which is not sticky, and a finish which is not shiny can be obtained. As a result, it becomes possible to suppress perspiration and fluid movement of the base material, and to maintain stability. The content of the non volatile oil is more preferably within a range from 20 to 60% by mass, and still more preferably within a range from 30 to 60% by mass. In the case that the transparency of the outer appearance is to be enhanced, a liquid oil component having a refractive index within a range from 1.4 to 1.6 is more preferable. The refractive index of the oil component is a value which is measured with a digital refractometer (Type: RX 5000 α by ATAGO), and examples thereof include dimethylpolysiloxane (approximately 1.40), liquid paraffin (approximately 1.47), etc.

Examples of hydrocarbon oils include liquid paraffin, ozokerite, squalane, pristane, paraffin, squalane, Vaseline, etc.

Examples of the ester oil include: isopropyl myristate, cetyl octanoate, octyl dodecyl myristate, isopropyl palmitate, butyl stearate, hexyl laurate, myristyl myristate, decyl oleate, hexyl decyl dimethyl octanoate, cetyl lactate, lactate myristyl, lanolin acetate, isocetyl stearate, isocetyl isostearate, cholesteryl 12 - hydroxy stearate, ethylene glycol di - 2 - ethyl hexanoate, dipenta erythritol fatty acid ester, N - alkyl glycol monoisostearate, neopentyl glycol dicaprate, diisostearyl malate, glycerin di - 2 - heptylundecanoate, trimethylolpropane tri - 2 - ethyl hexanoate, trimethylolpropane triisostearate, penta erythritol tetra - 2 - ethyl hexanoate, glycerin tri - 2 - ethyl hexanoate, glycerin trioctanoate, glycerin triisopalmitate, trimethylolpropane triisostearate, cetyl 2 - ethyl hexanoate, 2 - ethyl hexyl palmitate, glycerin trimyristate, glyceride tri 2 - heptylundecanoate, castor oil fatty acid methyl ester, oleyl oleate, acetoglyceride, 2 - heptylundecyl palmitate, diisobutyl adipate, N - lauroyl - L - glutamate - 2 - octyl dodecyl ester, di - 2-heptylundecyl adipate, ethyl laurate, di - 2 - ethyl hexyl sebacate, myristate 2- hexyl decyl, palmitate 2 - hexyl decyl, 2-hexyl decyl adipate, diisopropyl sebacate, 2 - ethyl hexyl succinate, triethyl citrate, etc.

Examples of the silicone oil include: linear polysiloxanes (for example, dimethyl polysiloxane, methyl phenyl polysiloxane, diphenyl polysiloxane, etc.), various modified polysiloxanes (amino modified polysiloxanes, polyether modified polysiloxanes, alkyl modified polysiloxanes, fluorine modified polysiloxanes, phenyl modified polysiloxanes, etc.), etc.

The following compounds may be employed as the ultraviolet ray absorbing agent.

### (1) Benzoate Ultraviolet Ray Absorbing Agents

For example, para amino benzoic acid (hereinafter abbreviated as PABA), PABA monoglycerin ester, N, N - dipropoxy PABA ethyl ester, N, N - diethoxy PABA ethyl ester, N, N - dimethyl PABA ethyl ester, N, N - dimethyl PABA butyl ester, N, N - dimethyl PABA ethyl ester, etc.

### (2) Anthranilate Ultraviolet Ray Absorbing Agents

For example, homomenthyl - N - acetyl anthranilate, etc.

### (3) Salicylate Ultraviolet Ray Absorbing Agents

For example, amyl salicylate, menthyl salicylate, homomenthyl salicylate, octyl salicylate, phenyl salicylate, benzyl salicylate, p - isopropanol phenyl salicylate, etc.

### (4) Cinnamate Ultraviolet Ray Absorbing Agents

For example, octyl cinnamate, ethyl - 4 - isopropyl cinnamate, methyl - 2, 5 - diisopropyl cinnamate, ethyl - 2, 4 - diisopropyl cinnamate, methyl - 2, 4 - diisopropyl cinnamate, propyl - p - methoxy cinnamate, isopropyl - p - methoxy cinnamate, isoamyl - p - methoxy cinnamate, octyl - p - methoxy cinnamate (2 - ethyl hexyl - p - methoxy cinnamate), 2 - methoxy ethyl - p - methoxy cinnamate, cyclohexyl - p - methoxy cinnamate, ethyl-α-cyano-β-phenyl cinnamate, 2-ethylhexyl-α-cyano-β-phenyl cinnamate, glyceryl mono-2-ethylhexanoyl-diparamethoxy cinnamate, etc.

### (5) Triazine Series Ultraviolet Ray Absorbing Agents

For example, bis resolcinyl triazine, etc.

More specifically, bis {[4-(2-ethylhexyloxy)-2-hydroxy]phenyl}-6-(4 -methoxyphenyl)1,3,5-triazine, 2,4,6-tris{4-(2-ethylhexyl oxy carbonyl) anilino} 1, 3, 5 - triazine, etc.

### (6) Other Ultraviolet Ray Absorbing Agents

For example, pyridazine derivatives such as 3-(4'-methylbenzylidene)-d, l-camphor, 3 - benzylidene - d, l-camphor, 2-phenyl-5-methylbenzoxazole,2,2'-hydroxy-5-methyl phenyl benzotriazole, 2 - (2' - hydroxy - 5' - t - octyl phenyl) benzotriazole, 2-(2'-hydroxy-5'-methyl phenyl benzotriazole, dianisoyl methane, 4 - methoxy-4'-t-butyl dibenzoyl methane, 5-(3,3-dimethyl-2-norbornylidene)-3 -pentane-2-on, and dimorpholino pyridazinone.

### (D) Silicone Resin Powder

A silicone powder having a refractive index within a range from 1.3 to 1.6 and an average particle diameter within a range from 0.1 to 30 µm, for example, the Tospearl Series by Momentive, the Silicone Powder KMP Series by Shin Etsu Chemical and polymethyl silsesquioxanes such as the MSP Series by Nikko Rika are favorable examples of the silicone resin powder. In addition, it is preferable for the silicone resin powder (d) to include a silicone rubber powder (d1). A silicone rubber powder (crosslinked polysiloxane) which is a cross polymer that includes dimethicone is more preferable. Examples of such silicone rubber powders include a (dimethicone/vinyl dimethicone) cross polymer, etc. Examples of commercially available products of silicone rubber powder (crosslinked polysiloxane) include the Trefil E Series and the COSMETIC POWDER Series by Toray Dow Corning, the KSP Series and the KSG Series by Shin Etsu Chemical, etc. The refractive index and the average particle size of the powder were determined with reference to documented values.

The amount of the silicone resin powder to be blended is within a range from 10 to 50% by mass, preferably a range from 12 to 48% by mass, more preferably a range from 15 to 40% by mass, and still more preferably a range from 8 to 35% by mass, with respect to the total amount of the composition. By setting the compounding amount of the silicone resin powder to be within a range from 10 to 50% by mass, it is possible to obtain a higher pore and unevenness correcting effect, and a better sensation of use. In addition, it is preferable for the oil content of the silicone rubber powder (d1) which is included in the silicone resin powder (d) to be within a range from 0.1 to 46% by mass with respect to 100% by mass of the oil based composition, more preferably a range from 0.5 to 40% by mass, and still more preferably a range from 1 to 30% by mass.

In addition, by setting the oil content of the silicone rubber powder (d1) which is included in the silicone resin powder (d) to be within the range of 0.1 to 46% by mass with respect to 100% by mass of the oil based composition, the oil based composition will be smooth with a melting sensation, and will change further after it adapts to the skin. In other words, the sensation of the texture of the oil based application changes by being applied to the skin, and a smooth translucent film can be formed. As a result, shadows due to unevenness of the skin will not be formed, pores can be made inconspicuous, and an appearance of transparency can be enhanced. In addition, because the oil based composition is smooth with a melting sensation when applied, it has flexibility, which results in a high sensation of adhesion to the skin. For this reason, the oil based composition becomes a clean finish without unstableness or thinning occurring when applied to the skin, and the amount of time that the cosmetic effect lasts will also become longer.

### (E) Spherical Powder

The oil based composition of the present invention may further contain a spherical powder other than the silicone resin powder.

Examples of the spherical powder include spherical silica, spherical nylon powder, spherical polyalkyl acrylate, cross linked polystyrene - squalane mixed powder, cellulose powder, anhydrous silicic acid powder, etc. The spherical powder may be nonporous or porous. In addition, the oil absorption of the spherical powder may be high or low. One or more types of the above spherical powders may be included in a range from 0.005 to 20% by mass with respect to 100% by mass of the oil based composition. A range from 0.005 to 15% by mass is preferable. The addition of the spherical powder helps to at least hide pores on the skin when utilizing the oil based composition, to adjust the texture, to improve adsorption of skin oils, and to further enhance a cosmetic lasting effect.

Other optional components can be blended in the oil based composition of the present invention, as long as the effects of the present invention are not impaired. Examples of such optional components include the solid oils and fats, waxes, powders, various oil soluble pharmaceutical agents, etc.

Examples of solid oils and fats include petroleum jelly, cocoa butter, coconut oil, hydrogenated coconut oil, palm oil, palm kernel oil, Japan wax kernel oil, hydrogenated oil, Japan wax, hydrogenated castor oil, etc.

Examples of waxes include ozokerite, beeswax, candelilla wax, cotton wax, carnauba wax, bayberry wax, privet wax, whale wax, montan wax, bran wax, lanolin, kapok wax, lanolin acetate, liquid lanolin, sugar cane wax, lanolin fatty acid isopropyl, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, POE hydrogenated lanolin alcohol ether, ceresin, microcrystalline wax, etc.

As the powder component, in addition to the spherical powders described above, an inorganic powder (for example, talc, kaolin, mica, sericite, muscovite, phlogopite, synthetic mica, flash mica, biotite, permiculite, magnesium carbonate, calcium carbonate, aluminum silicate, barium silicate, calcium silicate, magnesium silicate, strontium silicate, tungstenate metal salt, magnesium, silica, zeolite, barium sulfate, calcined calcium sulfate

(calcined gypsum), calcium phosphate, fluoroapatite, hydroxyapatite, ceramic powder, a metal soap (for example, zinc myristate, calcium palmitate, aluminum stearate), boron nitride, etc.); an inorganic white pigment (for example, titanium dioxide, zinc oxide, etc.); an inorganic red pigment (for example, iron oxide (colcothar), iron titanate, etc.); an inorganic brown pigment (for example, γ - iron oxide etc.); an inorganic yellow pigment (for example, yellow iron oxide, yellow earth, etc.); an inorganic black pigment (for example, black iron oxide, low order titanium oxide, etc.); an inorganic purple pigment (for example, manganese violet, cobalt violet, etc.); an inorganic green pigment (for example, chromium oxide, chromium hydroxide, cobalt titanate, etc.); an inorganic blue pigment (for example, ultramarine blue, bitumen, etc.); a pearl pigment (for example, titanium oxide coated mica, titanium oxide coated bismuth oxychloride, titanium oxide coated talc, colored titanium oxide coated mica, bismuth oxychloride, fish scale foil, etc.); a metal powder pigment (for example, aluminum powder, copper powder, etc.); an organic pigment such as zirconium, barium or aluminum lake (for example, an organic pigment such as red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404, Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3 and blue No. 1, etc); natural dyes (for example, chlorophyll, β carotene, etc.), etc.

Examples of liquid oils and fats include avocado oil, camellia oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cotton seed oil, perilla oil, soybean oil, peanut oil, tea seed oil, kaya oil, rice bran oil, Japanese oak oil, Japanese tung oil, jojoba oil, germ oil, triglycerin, etc.

Examples of lipophilic nonionic surfactants include sorbitan fatty acid esters (for example, sorbitan monooleate, sorbitan monoisostearate, sorbitan monolaurate, sorbitan monopalmitate, sorbitan monostearate, sorbitan sesquioleate, sorbitan trioleate, diglycerol sorbitan penta - 2 - ethyl hexyl acid, diglycerol sorbitan tetra - 2 - ethyl hexyl acid, etc.); glycerin poly glycerin fatty acids (for example, mono cotton seed oil fatty acid glycerin, mono erucic acid glycerin, sesquioleic acid glycerin, mono stearin acid glycerin, α, α' - oleic acid pyro glutamic acid glycerin, mono stearic acid glycerin malic acid, etc.); propylene glycol fatty acid esters (for example, propylene glycol monostearate, etc.); hydrogenated castor oil derivatives; glycerin alkyl ethers, etc.

Examples of hydrophilic nonionic surfactants include POE - sorbitan fatty acid esters (for example, POE - sorbitan monooleate, POE - sorbitan monostearate, POE - sorbitan monooleate, POE - sorbitan tetraoleate, etc.); POE sorbit fatty acid esters (for example, POE - sorbit monolaurate, POE - sorbit monooleate, POE - sorbit pentaoleate, POE - sorbit monostearate etc.); POE - glycerin fatty acid esters (for example, POE-monooleates such as POE - glycerin monostearate, POE - glycerin monoisostearate, POE - glyceryl triisostearate, etc.); POE - fatty acid esters (for example, POE - distearate, POE - mono dioleate, ethylene glycol distearate, etc.); POE - alkyl ethers (for example, POE - lauryl ether, POE - oleyl ether, POE - stearyl ether, POE - behenyl ether, POE - 2 - octyl dodecyl ether, POE - cholestanol ether, etc.); POE • POP - alkyl ethers (for example, POE • POP - cetyl ether, POE • POP 2 - decyl tetradecyl ether, POE • POP - monobutyl ether, POE • POP - hydrogenated lanolin, POE • POP-glycerin ether, etc.); POE - castor oil cured castor oil derivatives (for example, POE - castor oil, POE - hydrogenated castor oil, POE - hydrogenated castor oil monoisostearate, POE - hydrogenated castor oil triisostearate, POE - hydrogenated castor oil monopyroglutamic acid monoisostearic acid diester, POE - hydrogenated castor oil maleic acid, etc.); POE - beeswax lanolin derivatives (for example, POE - sorbit beeswax, etc.) alkanol amides (for example, coconut oil fatty acid diethanol amide, mono ethanol amide laurate, fatty acid isopropanol amide, etc.); POE - propylene glycol fatty acid ester; POE - alkylamine; POE - fatty acid amide; sucrose fatty acid ester; trioleyl phosphate, etc.

Examples of moisturizing agents include polyethylene glycol, propylene glycol, glycerin, 1, 3 - butylene glycol, xylitol, sorbitol, maltitol, chondroitin sulfate, hyaluronate, mucoitin sulfate, caroninate, atelocollagen, cholesteryl - 12 - hydroxy stearate, sodium lactate, bile salt, dl - pyrrolidone carboxylate, short chain soluble collagen, diglycerin (EO) PO adduct, rosa roxburghii extract, yarrow extract, melilot extract, trehalose, erythritol, POE • POP random copolymer methyl ether, etc.

Examples of metal ion sequestering agents include 1 - hydroxyethane - 1, 1 - diphosphonate, 1 - hydroxy ethane - 1, 1 - diphosphonate tetrasodium salt, edetate disodium, edetate trisodium, edetate tetrasodium, sodium citrate, sodium polyphosphate, sodium metaphosphate, gluconate, phosphate, citrate, ascorbate, succinate, edetate, trisodium ethylene diamine hydroxy ethyl triacetate, etc.

Examples of vitamins include vitamins A, B1, B2, B6, C, E and derivatives thereof, pantothenate and derivatives thereof, biotin, etc.

Examples of antioxidants include tocopherols, dibutyl hydroxy toluene, butyl hydroxy anisole, gallic acid esters, etc.

Examples of antioxidant aiding agents include phosphoric acid, citric acid, ascorbic acid, maleic acid, malonate, succinate, fumaric acid, kephalin, hexameta phosphate, phytic acid, ethylene diamine tetraacetic acid, etc.

Other components that can be added include, for example, preservatives (methyl paraben, ethyl paraben, butyl paraben, phenoxy ethanol, etc.); antifoaming agents (simethicone, etc.); anti inflammatory agents (for example, glycyrrhizinic acid derivatives, glycyrrhetinic acid derivative, thiotaurine, hypotaurine, hinokitiol, zinc oxide, allantoin, etc.); whitening agents (for example, saxifrage extract, albutin, tranexamic acid, L - ascorbic acid, magnesium salt of L - ascorbic acid phosphate ester, L - ascorbic acid glucoside, potassium 4 - methoxy salicylic acid, etc.); various extracts (for example, cork tree bark, coptis, gromwell root, peony, Japanese green gentian, birch, sage, loquat, carrot, aloe, mallow, iris, grape, coix seed, hemlock, lily, saffron, cnidium, ginger root, hypericum, restharrow, garlic, pepper, dried citrus peel, angelica, seaweed, etc.); activators (e.g., royal jelly, photosensitizer, cholesterol derivatives, etc.); blood circulation promoter, etc.

The oil based cosmetic composition of the present invention can be filled in a container after producing an oil based composition according to a conventional production method that includes dissolution, powder dispersion, mixing, etc. Particularly, because the amount of the volatile oil component which is included in the oil based composition of the present invention is slight, the container in which it is to be filled need not be airtight (for example, an ordinary metal dish without a lid), and the for thereof may be as an agent impregnated in a sponge, an agent packed in a tube or a bottle, etc. Versatility and cost savings are achieved for manufacturers, and a cosmetic which is convenient to utilize is provided to users. In addition, oil based cosmetic composition of the present invention can be used as a makeup cosmetic such as a foundation, a concealer, a cosmetic base, a highlighting blush, an eye shadow, etc.

### [Examples]

Next, the present invention will be described in greater detail with reference to Examples. Note that the present invention is not limited at all by the following examples. In addition, the amounts of components which are blended are indicated as % by mass unless otherwise noted.

The Examples and Comparative Examples were produced by a conventional method. Specifically, a gelling agent (dextrin fatty acid ester, etc.) was added to an oil and heated to dissolve at a temperature within a range from 80 to 90°C, and then the remaining components (ultraviolet ray absorbing agent, powder, etc.) were added and dispersed by a homomixer until the mixture became uniform. The compositions were degassed, filled into containers, and then cooled to room temperature, to obtain each of the oil based solid compositions.

### [Evaluations]

### [External Appearance]

The oil based solid compositions obtained in the Examples and the Comparative Examples were poured into white resin containers such that they had thicknesses of 7 mm, and the external appearances thereof were evaluated at room temperature.
A: The surface is smooth and there is no abnormality
B: Not transparent or white, discolored
C: Lumpy, cannot be poured and molded

### [Sensation of Use and Finish]

The samples obtained in Examples and Comparative Examples were applied, and sensory evaluations were performed by a panel of 20 specialists on sensation of smoothness, matte finish, uniformity of finish, and unevenness correcting effect. Evaluations were conducted as five scores and the average values of the scores given by the 20 panelists were judged by a four rank criteria.

### <Five Score Evaluation Criteria>

5: Very Good
4: Good
3: Fair
2: Somewhat Poor
1: Poor

### <Four Rank Criteria>

AA: 4 points or greater
A: 3 points or greater and less than 4 points
B: 2 points or greater and less than 3 points
C: Less than 2 points

The evaluation results are shown in Table 1 together with the compositions.

**[Table 1]**

| | Example 1 | Example 2 | Example 3 | Comparative Example 1 | Comparative Example 2 | Comparative Example 3 | Comparative Example 4 | Comparative Example 5 |
|---|---|---|---|---|---|---|---|---|
| Dextrin Palmitate | 8 | 8 | 8 | 8 | 8 | 8 | 8 | 8 |
| Liquid Paraffin | 14 | 14 | 14 | 14 | 14 | 14 | 14 | 14 |
| Isopropyl Myristate | 10 | 10 | 10 | 10 | 10 | 10 | 10 | 10 |
| Nonvolatile Dimethyl Polysiloxane | 19 | 19 | 19 | 12 | 19 | 19 | 19 | 19 |
| Octyl Methoxy Cinnamate | 3 | 3 | 3 | - | 3 | 3 | 3 | 3 |
| Plymethyl Silsesquiosane | 46 | - | 18 | - | - | - | - | - |
| (Dimethicone/Vinyl Dimethicone Crosspolymer | - | 46 | 15 | 56 | - | - | - | - |
| Spherical Anhydrous Silicic Acid (Nonporous) | - | - | - | - | 46 | - | - | - |
| Spherical Anhydrous Silicic Acid (Porous) | - | - | 12 | - | - | 46 | - | - |
| Spherical Polyurethane | - | - | - | - | - | - | 46 | - |
| Spherical Polyethylene | - | - | - | - | - | - | - | 46 |
| Amorphous Anhydrous Silica (High Oil Absorption) | - | - | 1 | - | - | - | - | - |
| Total | 100 | 100 | 100 | 100 | 100 | 100 | 100 | 100 |
| Outer Appearance | A | A | A | A | B Yellowing | C Lumpy* | C Lumpy* | C Lumpy* |
| Sensation of Smoothness | A | A | AA | A | A | A | A | C Gritty |
| Matte Finish | A | AA | A | AA | C | C | A | A |
| Uniform Finish | A | A | A | B Unstable | A | A | C Unstable | C Unstable |
| Unevenness Correcting Effect | A | AA | AA | AA | C | C | C | C |

As shown in Table 1, the oil based solid compositions of the Examples were superior in all of the appearance, the sensation of smoothness, the matte finish, the uniformity of finish, and the unevenness correcting effect.

In contrast, in Comparative Example 1 in which the amount of the silicone resin powder was great, instability occurred. In addition, in Comparative Examples 2 and 3, in which spherical anhydrous silicic acid was employed instead of the silicone resin powder, the unevenness correcting effect was insufficient regardless of whether the spherical anhydrous silicic acid was nonporous or porous, and matte finishes were not obtained. Further, yellowing was observed in the composition, and Comparative Example 3 became lumpy and could not be molded. Also in Comparative Example 4, in which spherical polyurethane was employed, the unevenness correcting effect was insufficient, instability was present, and this composition became lumpy and could not be molded. Comparative Example 5 as well, in which spherical polyethylene was used, the unevenness correcting effect was insufficient, instability and grittiness were present, and the composition became lumpy and could not be molded.

Subsequently, a comparison with the solid cosmetic disclosed in Patent Literature 1 (Examples 2 and 6) was also conducted (Comparative Examples 6 and 7). As Examples, Examples 4 and 5, in which the non volatile oil component of Example 3 was replaced with those Comparative Examples 6 and 7, were prepared. The results are shown in Table 2 together with the compositions. Note that neopentyl glycol dicaprate was substituted for glyceryl tri - 2 - ethyl hexanoate. As shown in Table 2, Comparative Examples 6 and 7 do not contain the silicone resin powder, have a high content of fine amorphous particles, and have a large content of non volatile oil components (methyl phenyl polysiloxane or neopentyl glycol dicaprate). For these reasons, a sensation of smoothness could not be realized, the unevenness correcting effect was insufficient, and matte finishes were not exhibited.

**[Table 2]**

| | Example 3 | Example 4 | Example 5 | Comparative Example 6 | Comparative Example 7 |
|---|---|---|---|---|---|
| Dextrin Palmitate | 8 | 8 | 8 | | 20 |
| Dextrin Myristate | | | | 20 | |
| Liquid Paraffin | 14 | | | | |
| Isopropyl Myristate | 10 | | | | |
| Methyl Phenyl Polysiloxane | | 46 | | 69 | |
| Neopentyl Glycol Dicaprate | | | 46 | | 65 |
| Nonvolatile Dimethyl Polysiloxane | 19 | | | | |
| Octyl Methoxy Cinnamate | 3 | | | | |
| Polymethyl Silsesquiosane | 18 | 18 | 18 | | |
| (Dimethicone/Vinyl Dimethicone Crosspolymer | 15 | 15 | 15 | | |
| Spherical Anhydrous Silicic Acid (Porous) | 12 | 12 | 12 | 4 | 10 |
| Amorphous Anhydrous Silica (High Oil Absorption) | 1 | 1 | 1 | 7 | 5 |
| Total | 100 | 100 | 100 | 100 | 100 |
| Sensation of Smoothness | A | A | A | C Sticky | C Sticky |
| Matte Finish | AA | A | A | C | C |
| Uniform Finish | A | A | A | A | A |
| Unevenness Correcting Effect | AA | A | A | C | C |

As is clear from the above examples, the oil based cosmetic of the present invention, even if it contains dextrin fatty acid ester, has usability as free flowing and is excellent in the unevenness correction effect. In addition, since the oil based composition of the present invention can be filled in a container having no air tightness, a container with high versatility and economy can be selected, and oil cosmetic that is easy for the user to use.

The following oil based cosmetic was produced by a conventional method.
Preparation Example 1: Foundation
   Dextrin fatty acid ester 8
   Liquid paraffin 14
   Isopropyl myristate 10
   Nonvolatile dimethyl polysiloxane 18
   Octyl methoxy cinnamate (octyl - p - methoxy cinnamate) 2
   Ethyl hexyl triazone 1
   Polymethyl silsesquioxane 18
   (Dimethicone/vinyl dimethicone) cross polymer 15
   Spherical anhydrous silica 12
   Amorphous anhydrous silica 1
   Antioxidant q. s.
   Moisturizer q. s.
   Antifoaming agent q. s.
Preparation Example 2: White powder
   Dextrin fatty acid ester 13.50
   Liquid paraffin 15
   Triethyl hexanoin 7.50
   Isopropyl myristate 9.50
   Nonvolatile dimethyl polysiloxane 17.50
   Bisbutyl dimethicone polyglyceryl-3 1
   Polymethyl silsesquioxane 15
   (Dimethicone/vinyl dimethicone) cross polymer 7
   Spherical anhydrous silica 12
   Amorphous anhydrous silica 1
   Titanium oxide q. s.
   Antioxidant, q. s.
   Moisturizer q. s.
   Fragrance q. s.
   Antifoaming agent q.s.
Preparation Example 3: Blush
   Dextrin fatty acid ester 13.50
   Liquid paraffin 22.50
   Triethyl hexanoin 8
   Diphenyl siloxy phenyl trimethicone 10
   Nonvolatile dimethyl polysiloxane 15
   Polymethyl silsesquioxane 15
   (Dimethicone/vinyl dimethicone) cross polymer 4
   Amorphous anhydrous silica 1
   Titanium oxide q. s.
   Iron oxide q. s.
   Red 226 1.50
   Mica 3
   Pearl agent 6
   Antioxidant, q. s.
   Moisturizer q. s.
   Fragrance q. s.
   Antifoaming agent q. s.
Preparation Example 4: Eye shadow
   Dextrin fatty acid ester 14
   Liquid paraffin 10
   Triethyl hexanoin 10
   Diphenyl siloxy phenyl trimethicone 10
   Nonvolatile dimethylpolysiloxane 10
   Polymethyl silsesquioxane 12
   (Dimethicone/vinyl dimethicone) cross polymer 1.50
   Amorphous anhydrous silica 1
   Iron oxide 12
   Pearl agent 13
   Antioxidant, q. s.
   Moisturizer q. s.
   Fragrance q. s.
   Antifoaming agent: q. s.

## Claims

1. An oil based composition comprising, with respect to 100% by mass of the oil based composition:
(a) 1 to 20% by mass of a dextrin fatty acid ester;
(b) less than 5% by mass of a volatile oil component having a boiling point of less than or equal to 250°C at normal pressure;
(c) 10 to 60% by mass of a non volatile oil which is a liquid at normal temperature and normal pressure; and
(d) 10 to 50% by mass of a silicone resin powder.

2. An oil based composition as defined in Claim 1, wherein:
the silicone resin powder (d) contains 0.1 to 46% by mass of a silicone rubber powder (d1) with respect to 100% by mass of the oil based composition.

3. An oil based composition as defined in Claim 2, wherein:
the silicone rubber powder (d1) is a cross polymer that includes dimethicone.

4. An oil based composition as defined in any one of Claims 1, 2, and 3, further comprising:
(e) a spherical powder.

5. An oil based cosmetic formed by filling a container which is not airtight with the oil based composition as defined in any one of Claims 1 through 4.
